# EUROPÄISCHE PATENTANMELDUNG

(11) **EP 3 964 152 A1**
(43) Veröffentlichungstag der Anmeldung: **09.03.2022**
(21) Anmeldenummer: 20194673.8
(22) Anmeldetag: 04.09.2020
(51) Int. Cl.: A61B 18/12, A61B 18/00

(54) **EINRICHTUNG ZUR GEWEBEBEHANDLUNG UND VERFAHREN ZUR ERFASSUNG EINES ELEKTRODEN/GEWEBE-KONTAKTS**

(71) Anmelder: Erbe Elektromedizin GmbH, 72072 Tübingen (DE)
(72) Erfinder: Keller, Sandra, 72379 Hechingen (DE); Blobel, Lars, 72119 Ammerbuch (DE)
(74) Vertreter: Rüger Abel Patentanwälte PartGmbB

(57) **Zusammenfassung**

Mit dem erfindungsgemäßen Konzept gelingt es, dem Operateur eine erhöhte Sicherheit zu vermitteln. Er kann sich darauf verlassen, dass eine Behandlungsspannung nur dann an das Gewebe (12) abgegeben wird, wenn das Instrument (11) korrekt in das Gewebe (12) eingeführt, beispielswiese eingestochen ist, sodass beide Elektroden (13, 14) Gewebekontakt haben. Bei unzureichender Sicht oder sonstigen Störfaktoren, die die visuelle oder anderweitige Kontrolle des korrekten Gewebekontakts der beiden Elektroden (13, 14) verhindern, wird auf diese Weise sicher eine Fehlaktivierung ausgeschlossen, bei der unzureichender Kontakt zwischen Elektrode und Gewebe zur Funkenbildung oder anderweitigen Schädigung von Gewebe führen könnte.

## Beschreibung

Die Erfindung betrifft eine Einrichtung zur Gewebebehandlung mittels eines elektrischen Instruments mit mindestens zwei Elektroden und ein Verfahren zur Erfassung des vollständigen Gewebekontakts mit mindestens zwei Elektroden.

Aus der WO 2017/048976 A1 ist ein elektrisches Instrument mit mehreren Schneiddrähten bekannt, die mit einer Wechselspannung gespeist sind, um biologisches Gewebe zu trennen. Während des Betriebs des Instruments wird der Leistungsfaktor überwacht, um zu ermitteln, ob an dem Schneiddraht Kurzschlussbedingungen oder die Bedingungen eines offenen Stromkreises vorliegen. Im Kurzschlussfalle wird dem Kreis ein weitgehend induktives Verhalten zugeschrieben, während dem offenen Stromkreis ein vorwiegend kapazitives Verhalten zugeschrieben wird. Der Leistungsfaktor wird während des Betriebs zur Leistungsanpassung herangezogen. Außerdem wird der Schneidvorgang als beendet angesehen, wenn der Leistungsfaktor Null ist.

Weiter ist aus der EP 2 612 612 A1 die Messung des Phasenwinkels der Impedanz zwischen der Elektrode des Instruments und der Neutralelektrode bekannt. Durch Vergleich des Phasenwinkels des Stroms mit einem Sollwert wird ein Abbruchkriterium für den Behandlungsvorgang hergeleitet.

Weiterer Stand der Technik wird durch die EP 2 475 319 B1**,** die US 5,514,129 B und, die DE 695 23 517 T2**,** die EP 2 552 335 B1 und die EP 2 301 463 A1 gebildet.

Bei elektrochirurgischen Instrumenten, insbesondere bei Instrumenten mit zwei oder mehreren Elektroden, kann es zur Durchführung einer korrekten Behandlung erforderlich sein, dass mindestens zwei oder auch mehrere Elektroden in physischer Berührung mit biologischem Gewebe stehen, beispielsweise indem eine entsprechende Sonde mit den Elektroden in das Gewebe eingestochen ist. Bei Behandlungen unter eingeschränkter Sicht, wie beispielsweise endoskopischen Behandlungen, kann es vor allem schwierig sein, zu verifizieren, ob tatsächlich zwei Elektroden sicheren Gewebekontakt haben.

Davon ausgehend ist es Aufgabe der Erfindung, eine elektrochirurgische Einrichtung zur elektrothermischen Behandlung von biologischem Gewebe zu schaffen, die sich mit erhöhter Sicherheit betreiben lässt.

Diese Aufgabe wird mit Einrichtung nach Anspruch 1 sowie auch mit dem Verfahren nach Anspruch 11 gelöst:

Die erfindungsgemäße elektrochirurgische Einrichtung umfasst ein Instrument, das mindestens zwei oder auch mehrere Elektroden aufweist, sowie ein Gerät zur Speisung dieses Instruments und seiner Elektroden. Das Instrument kann zwei oder mehrere Elektroden aufweisen. Zusätzlich kann es Elemente aufweisen, die z.B. zur Kühlung der Elektroden mit fluiden Medien, z.B. einem Kühlmedium zu speisen sind. Das Gerät kann darauf eingerichtet sein, solche Medien an das Instrument zu liefern.

Das Gerät enthält einen Generator zur Abgabe einer Behandlungsspannung und eine Steuereinrichtung zur Steuerung dieses Generators. Die Steuereinrichtung ist dabei so ausgebildet, dass sie entweder eine Testspannung oder eine Behandlungsspannung an das Gewebe abgeben kann. Die Testspannung ist dabei vorzugsweise eine Spannung, die an dem Gewebe keinen oder keinen wesentlichen chirurgischen Effekt bewirkt. Eine solche Testspannung liegt beispielsweise im Bereich von einigen Volt oder einigen 10 Volt.

Hingegen liegt die Behandlungsspannung typischer weise in einem Bereich von 100 Volt oder mehreren 100 Volt und ist geeignet, an oder in dem Gewebe einen Koagulationseffekt, einen Ablationseffekt oder einen anderen elektrochirurgischen Effekt hervorzurufen.

Erfindungsgemäß ist die Steuereinrichtung dazu eingerichtet, nach Empfang eines Aktivierungssignals eine Größe G zu bestimmen, die den Phasenwinkel phi zwischen der Spannung, zum Beispiel der Testspannung, und dem sich daraus ergebenden Strom charakterisiert. Die Größe G kann beispielsweise der Phasenwinkel selbst oder eine von dem Phasenwinkel linear oder nichtlinear abhängige Größe sein. Beispielsweise kann die Größe G der Leistungsfaktor LF (LF = cos phi) sein.

Gemäß der Erfindung ist außerdem ein Grenzwert G₂, der sogenannte "zweite Grenzwert", festgelegt, der demjenigen Phasenwinkel zugeordnet ist, der auftritt oder unterschritten wird, wenn beide Elektroden Gewebekontakt haben. Erfindungsgemäß wird der Generator nur dann zur Abgabe der Behandlungsspannung angesteuert, wenn sich die Steuereinrichtung im Aktivierungszustand befindet und zusätzlich die Größe G zwischen dem zweiten Grenzwert G₂ und einer Größe G₃ befindet, die für den Phasenwinkel gleich oder fast gleich Null charakteristisch ist. Die Größe G₃ ergibt sich bei einem Phasenwinkel, der auftritt, wenn zwischen den Elektroden eine Impedanz vorhanden ist, die im Wesentlichen (nur) von dem ohmschen Widerstand gebildet ist, der z.B. durch biologisches Gewebe gebildet ist. Mit anderen Worten der Betrag des ohmschen Widerstands des Gewebes ist sehr viel kleiner als der Betrag des von kapazitiven oder induktiven Einflüssen gebildeten Blindwiderstandes.

Den Aktivierungszustand nimmt die Steuereinrichtung so lange ein, wie das Aktivierungssignal vorliegt. Das Aktivierungssignal wird beispielsweise von einem Handschalter, einem Fußschalter oder einem sonstigen vom Operateur zu betätigenden Element abgegeben. Mit dieser Maßnahme wird sichergestellt, dass eine Aktivierung der mindestens zwei Elektroden, d.h. eine Belieferung derselben mit Behandlungsspannung, unterbleibt sofern nicht die Elektroden einen ausreichenden Gewebekontakt haben.

Die erfindungsgemäße Einrichtung überprüft nach Aktivierung zunächst mit einer Testspannung, ob zwischen den Elektroden vorwiegend ohmsches Verhalten, gemischt ohmsches-kapazitives Verhalten oder rein kapazitives Verhalten vorliegt. Ohmsches Verhalten wird einem Zustand zugeordnet, in dem beide beteiligten Elektroden Gewebekontakt haben. Gemischt ohmsches-kapazitives Verhalten ist typischerweise zu verzeichnen, wenn eine der Elektroden Gewebekontakt, die andere Elektrode jedoch keinen Gewebekontakt hat. Fast rein kapazitives Verhalten liegt vor, wenn keine der beiden Elektroden Gewebekontakt hat. Zur Erfassung der Kontaktsituation wird geprüft, ob die Größe G zwischen dem ersten Grenzwert G₁ und dem zweiten Grenzwert G₂ liegt. Ist dies der Fall, hat nur eine der beiden Elektroden Gewebekontakt. Liegt die Größe G außerhalb des Bereichs G₁, G₂ jenseits des ersten Grenzwerts G₁, ist kein Gewebekontakt vorhanden. Liegt Größe G hingegen außerhalb des Bereichs G₁, G₂ jenseits des zweiten Grenzwerts G₂ liegt sicherer Gewebekontakt beider Elektroden vor. Nur wenn diese Bedingung erfüllt ist, gibt die Steuereinrichtung die Abgabe der Behandlungsspannung durch den Generator frei und steuert diesen entsprechend.

Es ist vorteilhaft, wenn der erste Grenzwert G₁ für einen Phasenwinkel phi₁ in einem Bereich von 60° bis 80° festgelegt ist. Weiter ist es vorteilhaft, wenn der erste Grenzwert G₁ für einen Phasenwinkel phi₁ in einem Bereich von 65° bis 75° festgelegt ist. Vorzugsweise beträgt der Phasenwinkel phi₁ für den ersten Grenzwert G₁ 70°. Außerdem ist es vorteilhaft, wenn der zweite Grenzwert G₂ für einen Phasenwinkel phi₂ im Bereich von 40° bis 60° festgelegt ist. Weiter ist es vorteilhaft, wenn der zweite Grenzwert G₂ für einen Phasenwinkel phi₂ im Bereich von 45° bis 55° festgelegt ist. Vorzugsweise beträgt der Phasenwinkel phi₂ für den zweiten Grenzwert G₂ 50°.

Weitere Einzelheiten vorteilhafter Ausführungsformen der Erfindung sind Gegenstand der Ansprüche, sowie der Zeichnung und der zugehörigen Beschreibung. Es zeigen:

Figur 1 die erfindungsgemäße Einrichtung, in schematisierter Darstellung,

Figur 2 und 3 Kreisdiagramme zur Erläuterung der prinzipiellen Funktion der Einrichtung nach Figur 1.

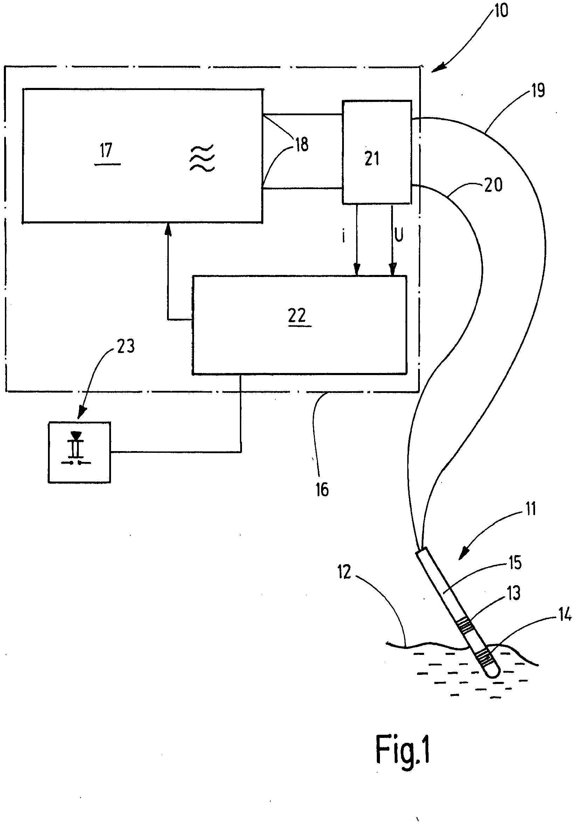

In Figur 1 ist eine elektrochirurgische Einrichtung 10 zur elektrothermischen Behandlung von biologischem Gewebe veranschaulicht. Die Einrichtung 10 kann beispielsweise eine Ablationseinrichtung, eine Koagulationseinrichtung oder irgendeine andere Einrichtung sein, die zur elektrothermischen Beeinflussung von biologischem Gewebe eingerichtet und/oder vorgesehen ist.

Zu der Einrichtung 10 gehört ein Instrument 11, das in direkte Wechselwirkung mit biologischem Gewebe 12 tritt. Das Instrument 11 kann als offenchirurgisches Instrument, als laparoskopisches Instrument, als endoskopisches Instrument, als flexible Sonde, usw. ausgebildet sein. Vorzugsweise ist es als bipolares Instrument mit mindestens zwei Elektroden 13, 14 ausgebildet, die zur Durchführung einer Behandlung beide mit dem Gewebe 12 in Berührung stehen müssen. Dazu sind die beiden Elektroden 13, 14 an einem elektrisch isolierenden Körper 15 des Instruments 11 angeordnet und zum Beispiel als Ringelektroden ausgebildet. Die Elektroden 13, 14 können aber beispielsweise auch Koagulationselektroden eines Zangeninstruments oder dergleichen sein.

Zum Betrieb des Instruments 11, insbesondere zur Speisung von dessen Elektroden 13, 14 mit Strom ist ein Gerät 16 vorgesehen. Dieses weist einen Generator 17 auf, der dazu eingerichtet ist, an seinem Ausgang 18 eine Spannung abzugeben. Vorzugsweise handelt es sich dabei um eine hochfrequente Wechselspannung mit einer Frequenz oberhalb 100 kHz und typischerweise unterhalb 10 MHz. Zum Beispiel liegt die Frequenz bei 350 kHz.

Die an dem Ausgang 18 anstehende Spannung kann entweder als Testspannung U_{T} geringe Werte von einigen Volt oder als Behandlungsspannung U_{HF} Werte von bis zu mehreren 100 Volt aufweisen.

Die von dem Generator 17 an dem Ausgang 18 abgegeben Spannung wird über Leitungen 19, 20 an die Elektroden 13, 14 geleitet. Ein zwischen den Leitungen 19, 20 und dem Ausgang 18 vorhandener Block 21, dient dabei sowohl dazu, die zwischen den Leitungen 19, 20 und somit zwischen den Elektroden 13, 14 vorhandene Spannung U als auch den in Leitungen 19, 20 fließenden Strom i zu erfassen und die entsprechenden Messwerte kontinuierlich oder von Zeit zu Zeit, beispielsweise zu gegebenen Abtastzeitpunkten, an eine Steuereinrichtung 22 zu melden. Die Steuereinrichtung 22 ist wiederum mit dem Generator 17 verbunden, um diesen entweder ein- oder auszuschalten oder zur Abgabe einer Testspannung U_{T} zu veranlassen oder ihn zur Abgabe einer Behandlungsspannung U_{HF} zu veranlassen.

Die Steuereinrichtung 22 wird mit einem Schalter 23 verbunden, der beispielsweise als Handschalter oder als Fußschalter ausgebildet ist und dazu dient, den Wunsch eines Behandlers zur Aktivierung des Generators 17 und des Instruments 11 zu erfassen. Außerdem ist die Steuereinrichtung 22 zumindest optional mit einer nicht veranschaulichten Anzeigeeinrichtung verbunden. Diese kann zum Beispiel eine akustische und/oder eine optische Anzeigeeinrichtung sein, die dazu eingerichtet ist, verschiedene Arbeitszustände zu signalisieren.

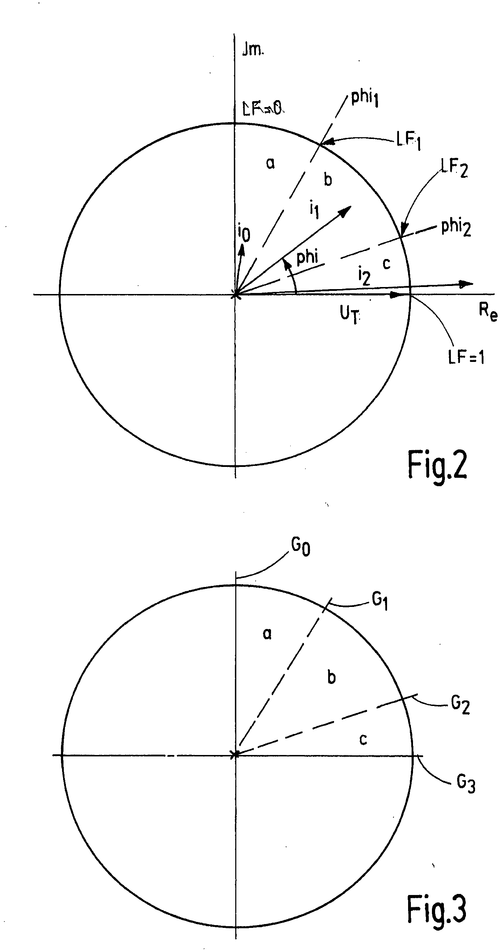

Die Steuereinrichtung 22 ist insbesondere dazu eingerichtet, den Phasenwinkel phi zwischen der Wechselspannung U und dem Wechselstrom i zu erfassen. Der Phasenwinkel phi ist davon abhängig, wie groß der Ohmsche Widerstand zwischen den Elektroden 13, 14 ist. Dazu wird auf Figur 2 verwiesen. Liegt beispielsweise eine Testspannung U_{T} an den Elektroden 13, 14 an, ergibt sich zwischen den Elektroden 13, 14 ein Strom i₀, i₁ oder i₂. Sowohl der Phasenwinkel phi als auch die Größe des Stroms sind davon abhängig, wie sich der Realteil und der Imaginärteil der Impedanz zwischen den Elektroden 13, 14 bzw. zwischen den Leitungen 19, 20 zueinander verhalten. Bei vollem Gewebekontakt beider Elektroden überwiegt der Realteil, d.h., der ohmsche Anteil der Impedanz. Bei teilweise oder ganz fehlendem Gewebekontakt einer oder beider Elektroden 13, 14 überwiegt der Imaginärteil, also liegt vorwiegend kapazitives Verhalten vor.

In Figur 2 steht der Strom i₀ für fehlenden Gewebekontakt, der Strom i₁ für einen Gewebekontakt lediglich mit der Elektrode 14 und der Strom i₂ für den Gewebekontakt beider Elektroden 13, 14. Für den Phasenwinkel phi können ein erster Grenzwert phi₁ und ein zweiter Grenzwert phi₂ festgelegt werden, sodass in einem Phasenwinkelbereich zwischen 90° und phi₁ fast rein kapazitives Verhalten zu vermerken ist, das für fehlenden Gewebekontakt beider Elektroden 13, 14 charakteristisch ist. Es kann ein zweiter Grenzwert phi₂ für den Phasenwinkel phi festgelegt werden, der, wenn er erreicht oder unterschritten ist, anzeigt, dass beide Elektroden 13, 14 Gewebekontakt haben. In dem Winkelbereich zwischen phi₁ und phi₂ hat lediglich eine der beiden Elektroden 13, 14 Gewebekontakt.

Bei einer einfachen Ausführungsform der Erfindung überwacht die Steuereinrichtung 22 den Phasenwinkel phi. Aktiviert der Chirurg oder ein sonstiger Benutzer den Schalter 23, geht die Steuereinrichtung 22 von ihrem Ruhezustand in einen Aktivierungszustand über, indem sie den Generator 17 zunächst veranlasst, an dem Ausgang 18 die Testspannung U_{T} abzugeben. Der Block 21 ermittelt nun den Phasenwinkel phi. Liegt dieser im Bereich zwischen 0° und dem zweiten Grenzwert phi₂, gibt die Steuereinrichtung 22 den Betrieb des Generators 17 mit Behandlungsspannung U_{HF} frei.

Bei einer einfachen Ausführungsform endet zu diesem Zeitpunkt die Phasenwinkelüberwachung. Es ist aber auch möglich, die Phasenwinkelüberwachung durch den Block 21 und die Steuereinrichtung 22 während der Behandlung fortzusetzen. In diesem Fall wird ständig oder von Zeit zu Zeit der Phasenwinkel phi zwischen der Behandlungsspannung U_{HF} und dem sich einstellenden Strom i ermittelt. Die Einrichtung 22 lässt die Zugabe der Behandlungsspannung U_{HF} solange zu, wie der Phasenwinkel phi in dem Bereich zwischen 0° und phi₂ liegt. Nimmt der Winkel phi jedoch zu, sodass er in den Bereich zwischen den beiden Grenzwerten phi₁ und phi₂ oder sogar zu noch größeren Werten wechselt, gibt die Steuereinrichtung 22 dem Generator 17 vor, die Abgabe der Behandlungsspannung U_{HF} zu beenden und stattdessen wieder die Testspannung U_{T} auszugeben.

In einer weiteren Ausführungsform kann die optional vorhandene optische und/oder akustische Anzeigeeinrichtung den Nutzer zusätzlich informieren, dass nur eine der Elektroden 13, 14 Gewebekontakt hat, sofern der Winkel phi zwischen den beiden Grenzwerten phi₁ und phi₂ liegt. Ebenso kann die Anzeigeeinrichtung den Nutzer zusätzlich informieren, dass keine der Elektroden 13, 14 Gewebekontakt hat, sofern der Winkel phi größer oder gleich dem Grenzwert phi₁ ist.

Bei dem vorstehend beschriebenen Ausführungsbeispiel überwacht die Steuereinrichtung 22 direkt den Phasenwinkel phi. Es ist aber auch möglich, anstelle des Phasenwinkels phi eine von dem Phasenwinkel phi abgeleitete Größe zu überwachen, wie beispielswiese den Leistungsfaktor LF. Der Leistungsfaktor LF ist gleich 1, wenn der Phasenwinkel phi gleich 0° ist. Der Leistungsfaktor LF ist gleich 0, wenn der Phasenwinkel phi gleich 90° ist. Üblicherweise wird der Leistungsfaktor LF berechnet als LF=cos(phi). Den beiden Phasenwinkelgrenzwerten phi₁ und phi₂ können entsprechende Leistungsfaktorgrenzwerte LF1, LF2 zugeordnet werden.

Bei einer solchen Ausführungsform bestimmt die Steuereinrichtung 22 den Leistungsfaktor und vergleicht ihn mit den beiden Leistungsfaktorgrenzwerten LF1 und LF2. Liegt der Leistungsfaktor zwischen den beiden Grenzwerten LF1 und LF2, steht lediglich eine der beiden Elektroden 13, 14 in Gewebekontakt. In diesem Fall wird, wenn die Aktivierung der Steuereinrichtung 22 fortgesetzt besteht, weiterhin die Testspannung U_{T} abgegeben. Liegt der Leistungsfaktor LF unterhalb des Grenzwerts LF1, liegt überhaupt kein Gewebekontakt vor, es wird wiederum nur die Testspannung U_{T} abgegeben. Ist der Leistungsfaktor LF jedoch größer als der zweite Leistungsfaktorgrenzwert LF2 wird die Abgabe der Behandlungsspannung U_{HF} freigegeben.

Die Erfindung ist nicht auf die Überwachung des Phasenwinkels phi oder des Leistungsfaktors LF beschränkt. Als zu überwachende Größe kann jede von dem Phasenwinkel phi abhängige Größe G dienen, sofern sie geeignet ist, die aus Figur 2 ersichtlichen drei Bereiche a, b und c zu unterscheiden. Der Bereich a steht für "keinen Gewebekontakt der Elektroden 13, 14". Der Bereich b steht für "Gewebekontakt einer der Elektroden 13, 14". Der Bereich c steht für den "Gewebekontakt beider Elektroden 13, 14".

Die Größe G kann beispielsweise eine linear von dem Phasenwinkel phi abhängige Größe oder eine nichtlinear von dem Phasenwinkel phi abhängige Größe sein. Beispielsweise kann die Größe G von einer Potenz des Phasenwinkels phi, z.B. der zweiten oder dritten Potenz desselben abhängen oder auch anderweitig von diesem abhängig sein.

Liegt bei Betätigung des Schalters 23 und Abgabe der Testspannung U_{T} an die Elektroden 13, 14 die Größe G beispielsweise in dem Bereich b, wird weiterhin nur die Testspannung U_{T} abgegeben. Liegt die Größe G jedoch außerhalb des Bereichs b in dem Bereich a zwischen dem Grenzwert G₁ und einem Grenzwert G₀, der für Phasenwinkel von 90° charakteristisch ist, wird ebenfalls lediglich Testspannung U_{T} freigegeben. Liegt der Wert der Größe G jedoch in dem Bereich c zwischen dem zweiten Grenzwert G2 und dem dritten Wert G3, der für einen Phasenwinkel phi von 0° charakteristisch ist, wird die Beaufschlagung der Elektroden 13, 14 mit Behandlungsspannung U_{HF} freigegeben, veranschaulicht in Figur 3.

Mit dem erfindungsgemäßen Konzept gelingt es, dem Benutzer eine erhöhte Sicherheit zu vermitteln. Er kann sich darauf verlassen, dass eine Behandlungsspannung nur dann an das Gewebe 12 abgegeben wird, wenn das Instrument 11 korrekten Kontakt mit dem zu behandelndem Gewebe 12 hat, bspw. es korrekt in das Gewebe 12 eingeführt, beispielswiese eingestochen ist, sodass beide Elektroden 13, 14 Gewebekontakt haben. Bei unzureichender Sicht oder sonstigen Störfaktoren, die die visuelle oder anderweitige Kontrolle des korrekten Gewebekontakts der beiden Elektroden 13, 14 verhindern, wird auf diese Weise sicher eine Fehlaktivierung ausgeschlossen, bei der unzureichender Kontakt zwischen Elektrode und Gewebe zur Funkenbildung oder anderweitigen Schädigung von Gewebe führen könnte.

### Bezugszeichen:

- 10: Einrichtung
- 11: Instrument
- 12: Gewebe
- 13, 14: Elektroden
- 15: elektrisch isolierender Körper des Instruments 11
- 16: Gerät
- 17: Generator
- 18: Ausgang des Generators
- 19, 20: Leitungen
- 21: Block
- U: Spannung, z.B. U_{HF} oder U_{T}
- i: Strom
- 22: Steuereinrichtung
- 23: Schalter
- LF: Leistungsfaktor
- phi: Phasenwinkel zwischen Spannung U und Strom i
- G: Größe für einen Grenzwert, z. B. G₀ oder G₁ oder G₂ oder G₃
- a,b,c: Auswertebereiche

## Patentansprüche

1. Elektrochirurgische Einrichtung (10) zur elektrothermischen Behandlung von biologischem Gewebe,
mit einem Instrument (11), das eine erste Elektrode (13) und mindestens eine zweite Elektrode (14) aufweist, die mit einer Testspannung (U_{T}) und mit einer Behandlungsspannung (U_{HF}) versorgbar sind,
mit einem Gerät (16), das einen Generator (17) zur Speisung des Instruments (11) mit der Testspannung (U_{T}) sowie mit der Behandlungsspannung (U_{HF}) aufweist,
mit einer Steuereinrichtung (22), die dazu eingerichtet ist, den Generator (17) zu veranlassen entweder die Testspannung (U_{T}) oder die Behandlungsspannung (U_{HF}) an das Instrument (11) abzugeben,
wobei die Steuereinrichtung (22) weiter dazu eingerichtet ist, nach Aktivierung in einen Aktivierungszustand zu schalten, im Aktivierungszustand den Generator (17) zunächst zu veranlassen, die Testspannung (U_{T}) an das Instrument (11) abzugeben und dabei eine Größe (G) zu bestimmen, die von dem Phasenwinkel (phi) eines sich ergebenden Stroms (i) in Relation zu der Testspannung (U_{T}) abhängig ist,
wobei die Steuereinrichtung (22) weiter dazu eingerichtet ist, den Generator (17) nur dann zu veranlassen, die Behandlungsspannung (U_{HF}) an das Instrument (11) zu liefern, wenn sich die Steuereinrichtung:
a) im Aktivierungszustand befindet und wenn außerdem
b) die Größe (G) in einem Bereich zwischen einem zweiten Grenzwert (G₂) und einer Größe (G₃) liegt, die für den Phasenwinkel (phi) gleich Null Grad charakteristisch ist.

2. Einrichtung nach Anspruch 1, **dadurch gekennzeichnet, dass** die Größe (G) der Phasenwinkel (phi) und der zweite Grenzwert (G₂) ein Phasenwinkelgrenzwert (phi₂) ist.

3. Einrichtung nach Anspruch 1, **dadurch gekennzeichnet, dass** die Größe (G) der Leistungsfaktor (LF) und der zweite Grenzwert (LF2) ein Leistungsfaktorgrenzwert ist.

4. Einrichtung nach einem der vorstehenden Ansprüche, **dadurch gekennzeichnet, dass** der zweite Grenzwert (LF2) einem Phasenwinkel (phi₂) entspricht, der präsent oder unterschritten ist, wenn sowohl die erste Elektrode (13) als auch die zweite Elektrode (14) Gewebekontakt haben.

5. Einrichtung nach einem der vorstehenden Ansprüche, **dadurch gekennzeichnet, dass** ein erster Grenzwert (G₁) festgelegt ist, der einem Phasenwinkel (phi₁) entspricht, der präsent oder unterschritten ist, wenn nur eine der beiden Elektroden (13, 14) Gewebekontakt hat.

6. Einrichtung nach einem der vorstehenden Ansprüche, **dadurch gekennzeichnet, dass** die Steuereinrichtung (22) derart ausgebildet ist, dass sie, wenn die Größe (G) einem Wert entspricht, der zwischen dem ersten Grenzwert (G1) und einer Größe (G₀) liegt, die für einen Phasenwinkel (phi) von 90° charakteristisch ist, ein Signal abgibt, das anzeigt, dass keine der beiden Elektroden (13, 14) Gewebekontakt hat.

7. Einrichtung nach einem der vorstehenden Ansprüche, **dadurch gekennzeichnet, dass** die Steuereinrichtung (22) derart ausgebildet ist, dass sie, wenn die Größe (G) zwischen dem ersten Grenzwert (G₁) und dem zweiten Grenzwert (G₂) liegt, ein Signal abgibt, das für fehlenden Gewebekontakt einer der Elektroden (13, 14) charakteristisch ist.

8. Einrichtung nach Anspruch 6, **dadurch gekennzeichnet, dass** die Steuereinrichtung (22) das Signal für den fehlenden Gewebekontakt beider Elektroden nur abgibt, wenn sie sich im Aktivierungszustand befindet.

9. Einrichtung nach Anspruch 7, **dadurch gekennzeichnet, dass** die Steuereinrichtung (22) das Signal für Gewebekontakt nur einer der beiden Elektroden (13, 14) nur abgibt, wenn sie sich im Aktivierungszustand befindet.

10. Einrichtung nach Anspruch 5, **dadurch gekennzeichnet, dass** der erste Grenzwert (G₁) für einen Phasenwinkel (phi₁) in einem Bereich von 60° bis 80° und der zweite Grenzwert (G₂) für einen Phasenwinkel (phi₂) im Bereich von 40° bis 60° festgelegt ist.

11. Verfahren zur Steuerung einer Einrichtung zur elektrothermischen Behandlung von biologischem Gewebe (12) mittels einer Steuereinrichtung (22),
bei dem die Steuereinrichtung (22) nach Empfang eines Aktivierungssignals in einen Aktivierungszustand schaltet und im Aktivierungszustand zunächst einen Generator (17) veranlasst, eine Testspannung (U_{T}) an das Instrument (11) abzugeben und dabei eine Größe (G) zu erfassen, die von dem Phasenwinkel (phi) eines sich ergebenden Stroms (i) in Relation zu der Testspannung (U_{T}) abhängig ist,
wobei die Steuereinrichtung (22) den Generator (17) nur dann veranlasst, die Behandlungsspannung (U_{HF}) an das Instrument (11) zu liefern, wenn sie sich
a) im Aktivierungszustand befindet und wenn
b) die Größe (G) in einem Bereich zwischen einem zweiten Grenzwert (G₂) und einer Größe (G₃) liegt, die für den Phasenwinkel (phi) gleich Null Grad charakteristisch ist.

12. Verfahren nach Anspruch 11, **dadurch gekennzeichnet, dass** die Steuereinrichtung (22) in einer Voraktivierungsbetriebsart den Generator (17) veranlasst, die Testspannung (U_{T}) an das Instrument (11) abzugeben und die Größe (G) zu ermitteln und diese mit dem ersten Grenzwert (G₁) und dem zweiten Grenzwert (G₂) zu vergleichen und, um daraus ein Signal abzuleiten, das anzeigt, dass nur eine Elektrode (13) Gewebekontakt hat, wenn der die Größe (G) zwischen den beiden Grenzwerten (G₁, G₂) liegt, und das anzeigt, dass beide Elektroden (13, 14) Gewebekontakt haben, wenn die Größe (G) zwischen dem zweiten Grenzwert (G₂) und einer Größe (G₃) liegt, die für den Phasenwinkel (phi) gleich Null Grad charakteristisch ist.
